# EUROPEAN PATENT APPLICATION

(11) **EP 1 811 301 A1**
(43) Date of publication of application: **25.07.2007**
(21) Application number: 05793178.4
(22) Date of filing: 13.10.2005
(51) Int. Cl.: G01N 33/543, G01N 21/27, G01N 21/64

(54) **METHOD OF INTERACTION OBSERVATION**

(30) Priority: 20.10.2004 JP 2004305605
(71) Applicant: Riken, Wako-shi, Saitama 351-0198 (JP); Toyo Boseki Kabushiki Kaisha, Osaka-shi, Osaka 530-8230 (JP)
(72) Inventor: KANOH, Naoki, Wako-shi, Saitama 3510198 (JP); OSADA, Hiroyuki, Wako-shi, Saitama 3510198 (JP); KYO, Motoki, Osaka-shi, Osaka 5308230 (JP)
(74) Representative: Müller-Boré & Partner Patentanwälte
(86) International application number: PCT/JP2005/018841
(87) International publication number: WO 2006/043461

(57) **Abstract**

A method of label-free observing of any interaction between biomolecule and substance immobilized on a metal substrate, **characterized in that** the substance is immobilized on the metal substrate by a covalent bond through photoreaction.

## Description

### Technical Field

The present invention relates to a method in which a substance is immobilized to a metal substrate by a photoreaction and an interaction of the immobilized substance with a biomolecule is analyzed.

### Background Art

As a procedure to analyze a function of a biomolecule in vivo, interactions of the biomolecules have been examined. As one of the procedures to examine the interactions, a method in which one molecule (A) in a binding pair is immobilized to a solid surface and the interaction with a molecule (B) subjected to the measurement is analyzed has been generally used.

When the biomolecule is immobilized, the immobilization method has been also investigated. As the method for immobilization, physical absorptions, chelate bonds, covalent bonds, ionic bonds and hydrogen bonds have been used. It is known from those findings that the procedure to immobilize the biomolecule is important. Because when the biomolecule is immobilized to the surface via functional group present in an active site (binding site) of the biomolecule, the biomolecule can not keep its activity.

As the method of generally controlling the immobilization, a functional group or the groups are previously introduced in a terminus or a site not involved in the activity of the biomolecule, and the biomolecule is immobilized on the surface via the functional group or the groups. However, when the functional group or the groups can not be introduced in the terminus of the site not involved in the activity, the conventional method can not be applied. In particular, smaller molecular substances which are drugs or drug candidates or cyclic substances which have no terminus are difficult to be immobilized.

Recently, Kanoh et al. have proposed the method of producing arrays on which small molecules have been immobilized by a photoreaction not depending on the functional group (Non-patent literature 1). In this method, since the small molecules are immobilized in a random direction, it is predicted that a part of the small molecules are certainly immobilized in an effective direction. Thus, it is possible to screen the small molecule having the interaction with a target molecule.

However, in this method, the small molecule is immobilized on a glass slide. To know whether the target substance interacted or not, it is necessary that the target substance has been labeled with a fluorescent molecule, or that the target substance is detected using a fluorescence labeled antibody. It is a complicated manipulation to label the target molecule. When the target molecule is a protein, it is sometimes necessary to express as a fusion protein by incorporating GFP (green fluorescent protein) for example in an expression vector for the protein. When the fluorescence labeled antibody is used, if the target substance is not common, it is necessary to produce its antibody.

Therefore, if the interaction of the immobilized small molecule with the target substance can be observed with label free and with real-time, this is very preferable, but such an invention has not been made yet.

In Patent document 1, the method in which the interaction of the small molecule immobilized to a vessel bottom with the target substance is observed with label free using surface plasmon resonance is described. However, the method of immobilizing the small molecule is ordinary, no method using light is shown, it is presumed that the functional group is used to immobilize, and thus, it is hardly thought that the correct interaction can be observed.

Conventionally, the method of immobilizing the substance to the gold surface via a photoreaction group was not used. This is supposed to be because a gold-sulfur bond is broken by the light (Non-patent literature 2). In the literature 2, it is described that oxidation by UV light at 254 nm by oxygen is required for breaking the gold-sulfur bond. In the immobilization to the gold surface by the photoreaction, the substance is not immobilized, and this method is fraught with risk that the gold is exposed. Patent document: JP 2004-271188-A
Non-patent literature 1: Angew. Chem. Int. Ed., 42 (2003) 5584-5587
Non-patent literature 2: J. Am. Chem. Soc., 123 (2001) 4089-4090.

This invention enables the immobilization method of the substance to the metal substrate by the photoreaction and enables to observe the interaction of the biomolecule with the immobilized substance.

### DISCLOSURE OF THE INVENTION

### PROBLEMS SOLVED BY THE INVENTION

An object of the present invention is to provide a method in which a substance is immobilized to a metal substrate surface by a photoreaction and the interaction of the immobilized substance with a biomolecule can be observed.

### MEANS FOR SOLVING THE PROBLEMS

As a result of an extensive study, the present inventors have found that the above problems can be solved by the procedure shown below.
[1] A method of label-free observing an interaction, characterized in that a substance is immobilized on a metal substrate via a covalent bond by a photoreaction in a method of label-free observing an interaction of a biomolecule with the substance immobilized on the metal substrate.
[2] The method according to [1] above wherein the photoreaction is performed using light with a wavelength of 300 nm or more.
[3] The method according to any of [1] to [2] above wherein a method of observing the interaction is a surface plasmon resonance.
[4] The method according to any of [1] to [2] above wherein a method of observing the interaction is a surface plasmon resonance imaging.
[5] The method according to any of [1] to [4] above wherein multiple substances are immobilized.
[6] The method according to any of [1] to [5] above wherein the immobilized substance is a drug or a drug candidate substance.
[7] The method according to any of [1] to [6] above wherein a molecular weight of the immobilized substance is 2,000 or less.
[8] The method according to any of [5] to [7] above wherein the biomolecule is a protein.

### BRIEF DESCRIPTION OF THE DRAWINGS

FIG. 1 shows a chemical structural formula of a photolinker;
FIG. 2 shows a chemical structural formula of a dummy linker;
FIG. 3 shows a gold surface on which the photolinker and the dummy linker have been introduced;
FIG. 4 shows a chemical structural formula of cyclosporin A;
FIG. 5 shows a chemical structural formula of digoxin;
FIG. 6 shows a chemical structural formula of digitoxin;
FIG. 7 shows a chemical structural formula of Digoxigenin;
FIG. 8 shows a chemical structural formula of β-estradiol;
FIG. 9 shows a chemical structural formula of hydrocortisone;
FIG. 10 shows a chemical structural formula of progesterone;
FIG. 11 shows results of observing an interaction with anti-CsA antibody (Example);
FIG. 12 shows results of observing an interaction with anti-DIG antibody (Example);
FIG. 13 shows results of observing an interaction with anti-EST antibody (Example);
FIG. 14 shows results of observing an interaction with anti-CsA antibody (Comparative Example 1); and
FIG. 15 shows results of observing an interaction with anti-DIG antibody (Comparative Example 2).

### BEST MODES FOR CARRYING OUT THE INVENTION

The present invention will be described in detail below. The present invention discloses a method of immobilizing a substance on a metal substrate by a photoreaction and a method of observing an interaction of a biomolecule with the immobilized substance.

The method of immobilizing the substance on the metal substrate by the photoreaction can be carried out, for example, by the method described below.

That is, the immobilization of the substance on the metal substrate can be carried out by the method including
(1) a step of contacting a solution containing the substance with the metal substrate where a photoreactive compound has been bound to its surface;
(2) a step of drying the solution containing the substance in contact with the metal substrate if necessary; and
(3) a step of irradiating the light to the metal substrate to form a covalent bond between the photoreactive compound and the substance.

In the present invention, the substance is immobilized on the substrate directly or via an appropriate spacer, and the interaction of the biomolecule which is a target substance with the immobilized substance is observed. By analyzing the interaction, it is possible to analyze the property of the immobilized substance or the biomolecule, and its numerous contribution to drug discovery and basic study in molecular biology can be expected. The substance immobilized here is immobilized on the substrate via the covalent bond by the photoreaction with a photoreactive functional group introduced on the substrate surface. There are two reasons why the photoreaction is used. (1) The photoreaction progresses with generating radical. Thus, the photoreactive group can be reacted with various functional groups and in some cases the reaction progresses not depending on the functional group. As a result, the substance can be immobilized in the random direction. Among the immobilized substances, a portion thereof is immobilized in a correct direction. Thus, it is highly likely that the interaction can be observed. (2) It is possible to irradiate the light to only a region to be reacted. Thus, free design of chips becomes possible.

The photoreactive compound is not particularly limited as long as it can be activated by light irradiation and form the covalent bond with a small molecular compound, and can include, for example, the following compounds of (a) to (c).
(a) Compounds capable of generating nitrene, carbene, radical or carbon electrophilic agent
   Compounds capable of generating these active species have been described in JP 2001-178472-A; Review written by S. A. Fleming in Tetrahedron 51:12479-12520, 1995; and Review written by Y. Hatanaka in Journal of Synthetic Organic Chemistry, Japan 56:581-590, 1998. For example, the compounds which generate nitrene are compounds such as aromatic azide, alkyl azide and heterocyclic azide having an azide group. The compounds which generate carbene are the compounds having a diazo group or a diazirine group. The compounds which generate radical are conjugate ketones such as benzophenones and enones, aromatic halogens and olefins. The compounds which generate the carbon electrophilic agent are aromatic diazonium salts, nitrobenzenes, sulfonium salts, phosphonium salts and ammonium salts.
(b) Compounds containing diazonium group, azide group, diazirine ring or diazo group as a partial structure
   The compounds having these partial structures have been also described in JP 2001-178472-A described above.
(c) Compounds represented by a formula (I): 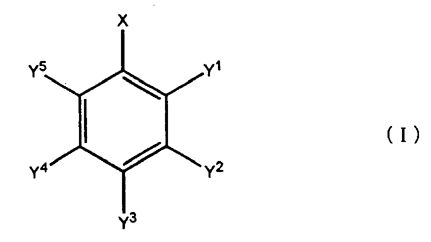
wherein X represents -N₃, -C*(R¹)N=N* (* are linked together to form a 3-membered ring), -N₂⁺Z⁻, -C(R²)=O, -CH=CH₂, -NO₂, -NH₂, - C(=O)N₃, -Cl, or -NH-CH₂-CO-CH=N₂; R¹ represents a hydrogen atom, an alkyl group which may have substituents or an aryl group which may have substituents; R² represents an aryl group which may have substituents; Z⁻ represents anion; any one of Y¹, Y², Y³, Y⁴, and Y⁵ represents a group capable of forming the covalent bond by reacting with the functional group supported on a solid phase carrier surface and other four each independently represent hydrogen atoms or halogen atoms.

In the compound represented by the general formula (I), the group represented by R¹ is preferably an alkyl group having 1 to 6 carbon atoms which may have the substituents or phenyl group having 6 to 12 carbon atoms which may have the substituents, and particularly preferably the alkyl group substituted with an electron attractive group such as a fluorine atom. The group represented by R² is preferably phenyl group. The anion represented by Z⁻ is preferably halide ion, boron tetrafluoride ion or phosphate hexafluoride ion. The group capable of forming the covalent bond by reacting with the functional group supported on the solid phase carrier surface may be any of Y¹, Y², Y³, Y⁴, and Y⁵, but Y³ is preferable. As the group capable of forming the covalent bond by reacting with the functional group supported on the solid phase carrier surface, preferable are carboxyl, formyl, active ester, hydroxyl, thiol, sulfide, amino, halogen-substituted alkyl, trialkoxysilyl groups and groups having these substituents.

Specific examples of the compounds represented by the general formula (I) include, but are not limited to, the following compounds 1 to 27.

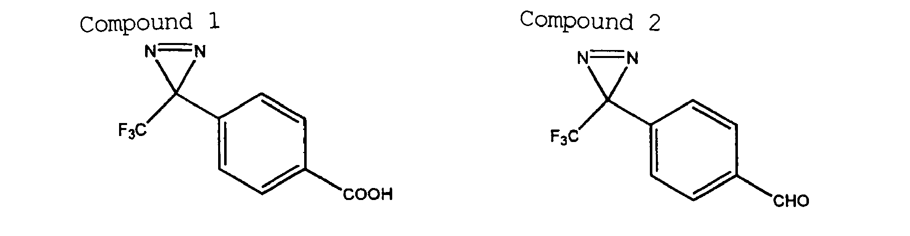

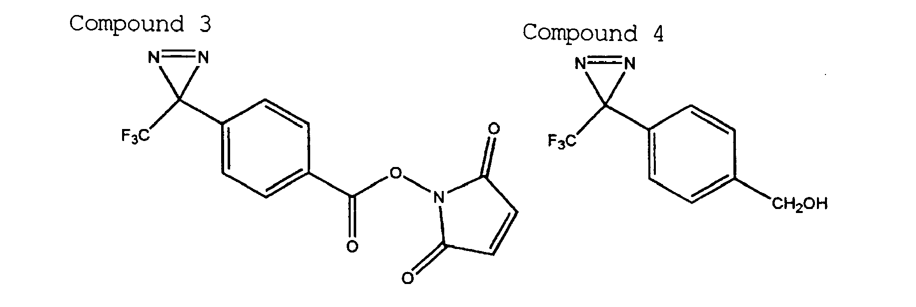

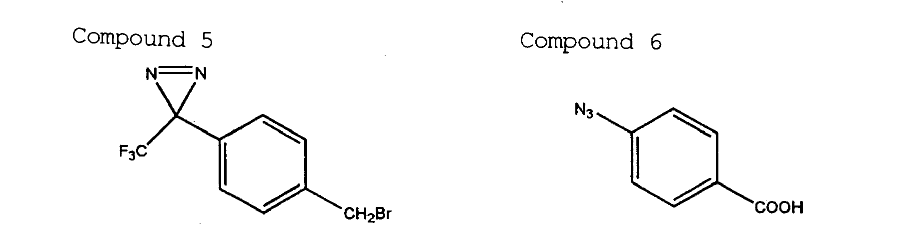

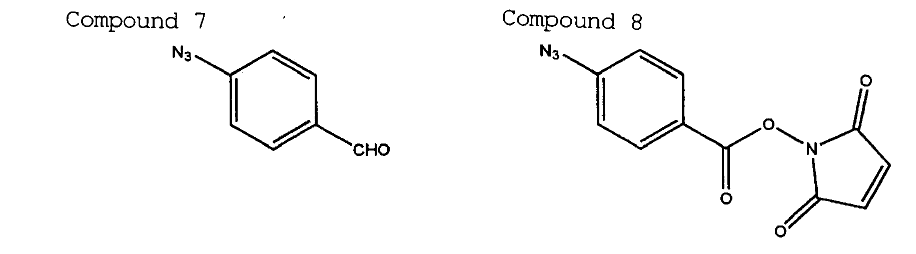

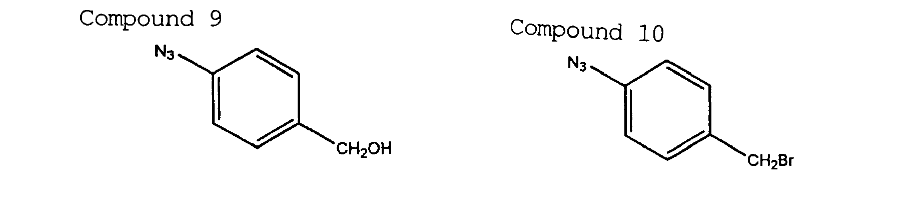

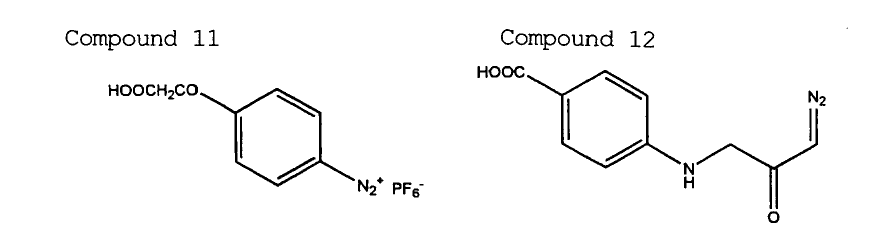

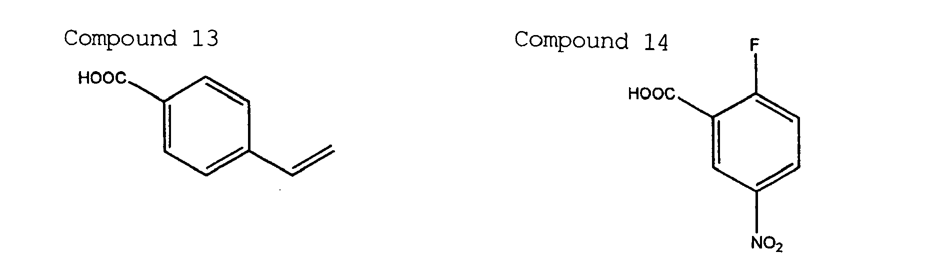

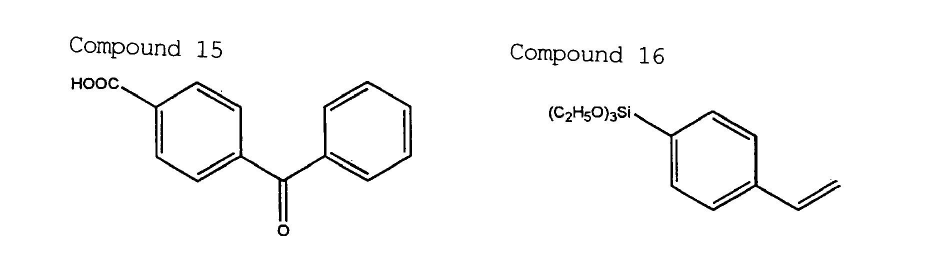

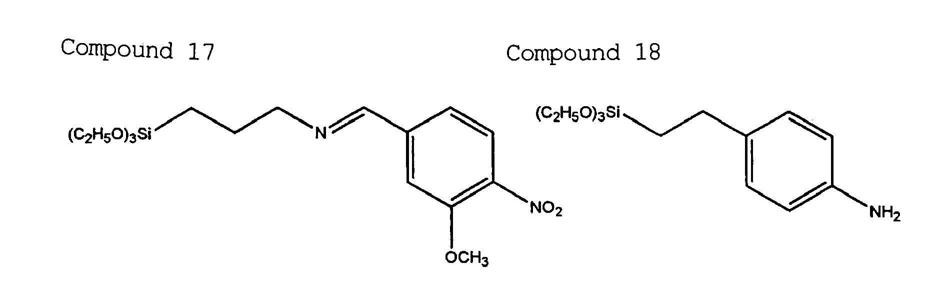

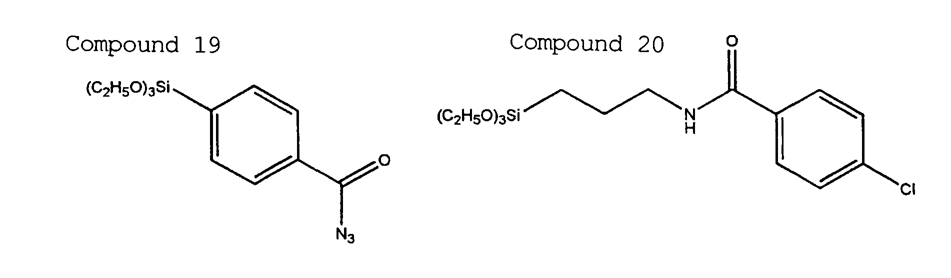

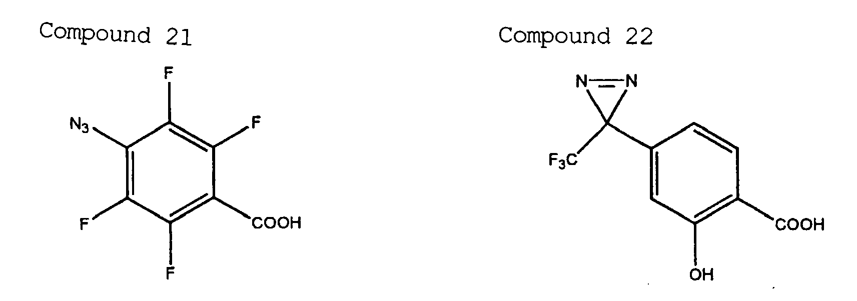

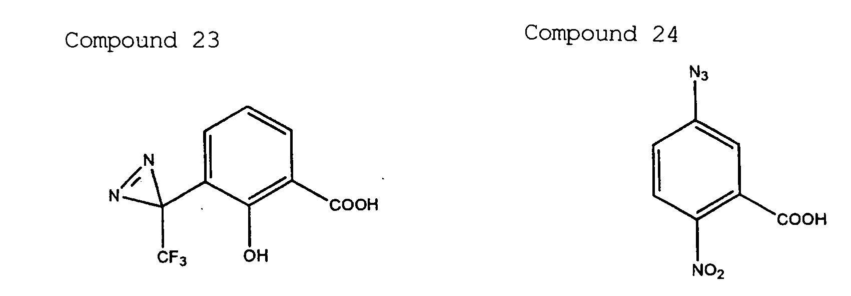

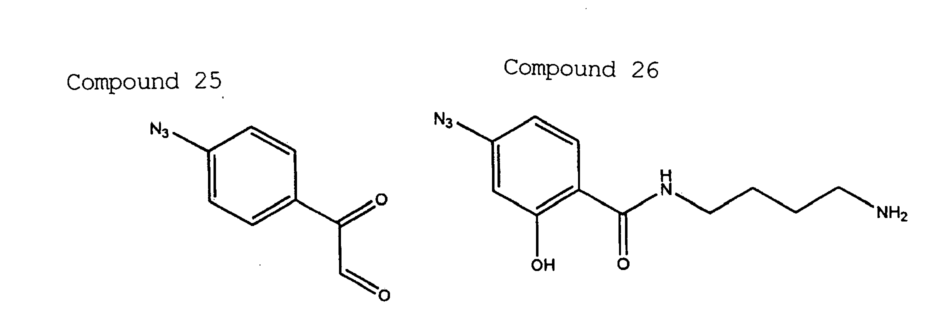

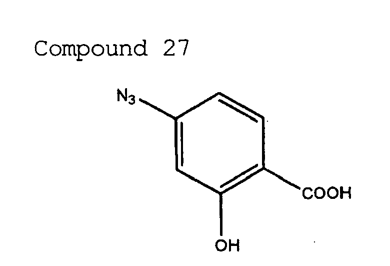

The immobilization method generally includes the method in which the solution of the substance to be immobilized is positioned on the metal substrate and the substance is immobilized via a covalent bond by reacting the substance with the photoreactive functional group introduced onto the substrate. Types of the solution are not particularly limited, and include water, buffers, and organic solvents such as dimethylsulfoxide (DMSO), N,N-dimethylformamide (DMF), dioxane, acetonitrile and chloroform.

As the substrate, the metal substrate is preferable. Because the surface is easily modified in the metal substrate. It is also advantageous in that thermal stability is excellent and drug resistance is high. The metal substrate includes not only plain faces but also particulate ones. Among them, gold is particularly preferable because the gold is excellent in stability and an optional functional group can be introduced onto the surface by forming a self-assembled monomolecular layer by alkane thiol having a thiol group at one terminus and a functional group capable of introducing the photoreactive compound (directly or via the spacer) at other terminus. The spacer such as polyethylene glycol may be present between the introduced photoreactive functional group and alkane thiol. It is also preferable to mix alkane thiol (photolinker) having the photoreactive functional group and alkane thiol (dummy linker) not having it and introduce them onto the surface. It can be anticipated that non-specific absorption is inhibited by the spacer and the dummy linker.

It is more preferable to use the metal substrate obtained by forming a thin film of the gold on the transparent substrate such as glass or plastic. Because, this can be applied to optical measurement methods. In this case, the method of forming the thin film of the gold is not particularly limited, and the method such as deposition, spattering or ion coating is selected.

The light used for the photoreaction in the immobilization preferably has a wavelength of 300 nm or more. When the wavelength is less than 300 nm, modification on the metal is broken and the immobilized substance is likely to be entirely dissociated. The metal is also exposed, leading to increased possibility of non-specific absorption which is not preferable. In particular, it is known that when the surface is modified on the gold thin film using alkane thiol by the gold-sulfur bond, sulfur is oxidized by the UV light of 254 nm and alkane thiol is dissociated (Non-patent literature 2). Therefore, when the photoreaction is performed using the UV light less than 300 nm, it is highly likely that the substance can not be immobilized, which is not preferable.

It is more preferable to use the light with a wavelength of 350 nm or more. It can be expected that the effect on the metal-sulfur bond is further reduced. But, when the light with a wavelength of 600 nm or more is irradiated, a reaction efficiency is very poor, which is not preferable.

A light source is not particularly limited, and sunlight, light of lamps such as mercury lamps, fluorescent lamps, xenon lamps and argon lamps, laser light (semiconductor laser, solid laser, gas laser), light from light emitting diode and light of electroluminescent elements can be utilized. As the method of irradiating the light, the light from the light source may be evenly irradiated to the solid phase carrier surface via an appropriate filter if necessary, or a pattern exposure of a desired shape may be performed using a so-called mask. Alternatively, the light may be concentrated using a lens or a mirror and irradiated in fine shape. Scanning exposure may be performed using the concentrated light. An irradiation time period is not particularly limited.

When multiple substances are introduced, for example, a first substance is applied onto the substrate to which the photoreactive functional group has been introduced, after drying as needed, the light is irradiated to a particular location to immobilize the first substance via the photoreactive functional group, and then the not immobilized first substance is washed out and removed.

Subsequently, a second substance is applied onto the substrate, the light is irradiated to an appropriate location where the first substance has not been immobilized (the light has not been irradiated) to immobilize the second substance, and then the non immobilized second substance is washed out and removed.

Hereinafter, by repeating this process, it is possible to immobilize the substances in a desired number on the chip with a desired pattern.

In the present invention, the interaction of the immobilized substance with the biomolecule which is the target molecule can be observed with label free. The label free refers to using no substance such as fluorescence or radioisotope required for the detection in the detection. It is highly likely that labeling with the fluorescence or the radioisotope denatures the target molecule, which is not preferable. There are also many molecules which are difficult to be labeled. Thus, being label free is extremely advantageous. If the observation can be performed with real time, it is more preferable. Because, by analyzing a binding speed and a dissociation speed, it is possible to obtain more information for the interaction between the immobilized substance and the target molecule.

Label free detection procedures include surface plasmon resonance (SPR), quartz crystal microbalance (QCM), ellipsometry, dual polarization interference, sum frequency generation (SFG) and second harmonic generation (SHG). Among them, SPR is preferable because multiple points can be measured simultaneously by optical operation. An SPR imaging method is the method in which p-polarized light flux is irradiated in the wide range of the chip and its reflection image is photographed by CCD camera, which is more preferable because the analysis in array format is possible.

Since multiple points can be measured in SPR, in the present invention, it is preferable that the multiple substances are immobilized. Because high throughput screening becomes possible by analyzing the multiple substances simultaneously. It is more preferable that the SPR analysis is performed using the array to which 5 or more substances have been immobilized.

The substance immobilized on the metal substrate by the photoreaction includes proteins, sugars, peptides, nucleic acids, lipids and organic compounds, and drugs or drug candidate substances are preferable. Here, the drug refers to the substance generally administered to patients as a medicament. Because the drug screening method is desired in many fields. More preferably, the drugs are organic compounds containing carbon, hydrogen, oxygen, nitrogen and sulfur atoms as major components, primary metabolites such as oligosaccharides and polypeptides, secondary metabolites such as fatty acids, polyketide (acetogenin), isoprenoid, phenyl propanoid and alkaloid, synthetic organic compounds with molecular weight up to 2,000 including aromatic rings and heterocycles, or complexes thereof, and have the possibility that the function of a protein is inhibited or facilitated by mainly binding to the protein.

The molecular weight of the substance immobilized by the photoreaction is preferably 2,000 or less. Because the substance having the molecular weight more than 2,000 is difficult in absorption in vivo and permeation of a cell membrane as the medicament. The molecular weight of the immobilized substance is preferably 1,500 or less and more preferably 1,000 or less. When the present invention is carried out using an array chip, the substance having the molecular weight more than 2,000 may be immobilized on the array, but it is preferable that at least one or more substances having the molecular weight of 2,000 or less are present.

The target substance which interacts with the immobilized substance includes proteins, sugars, peptides, nucleic acids, lipids and organic compounds, and among them, the protein is preferable. In particular, the present invention can exert the great effect on the method of screening small molecules which interact with the protein which is difficult to be labeled.

The solvent used for the preparation of a sample substance solution is not particularly limited, can be appropriately determined depending on the type of the sample substance, and for example, water, phosphate buffer, acetate buffer and tris buffer can be used. A concentration of the sample substance solution varies depending on the types of the small molecular compound and the solvent, and in the case of a buffer solution of the protein having the molecular weight of about 160,000, the concentration is about 0.1 to 200 µg/mL.

### EXAMPLES

The present invention will be specifically described with reference to the following Examples, but the present invention is not limited thereto.

### [Example 1]

A gold deposited chip was obtained by depositing 2 nm of chromium on a transparent glass substrate, SF15 with a thickness of 2 mm (18 mm x 18 mm) and subsequently depositing 45 nm of gold. The thickness of the deposition was monitored by quartz crystal microbalance.

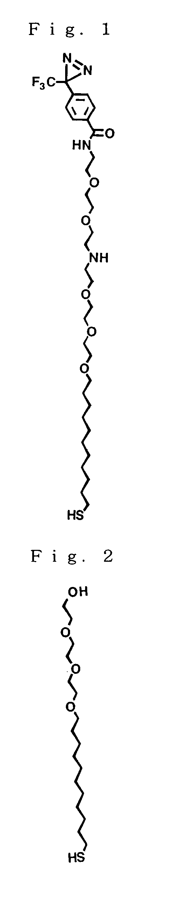

Alkane thiol having 4-{3-(trifluoromethyl)-3H-diazirin-3-yl}benzoyl group as a photoreactive group was used as a photolinker (FIG. 1). Polyethylene glycol has been inserted between the photoreactive group and alkane thiol.

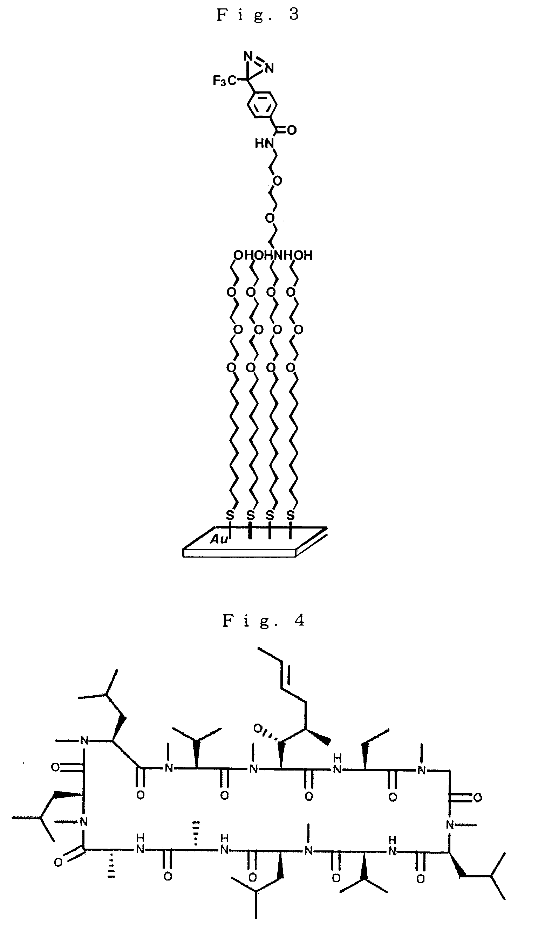

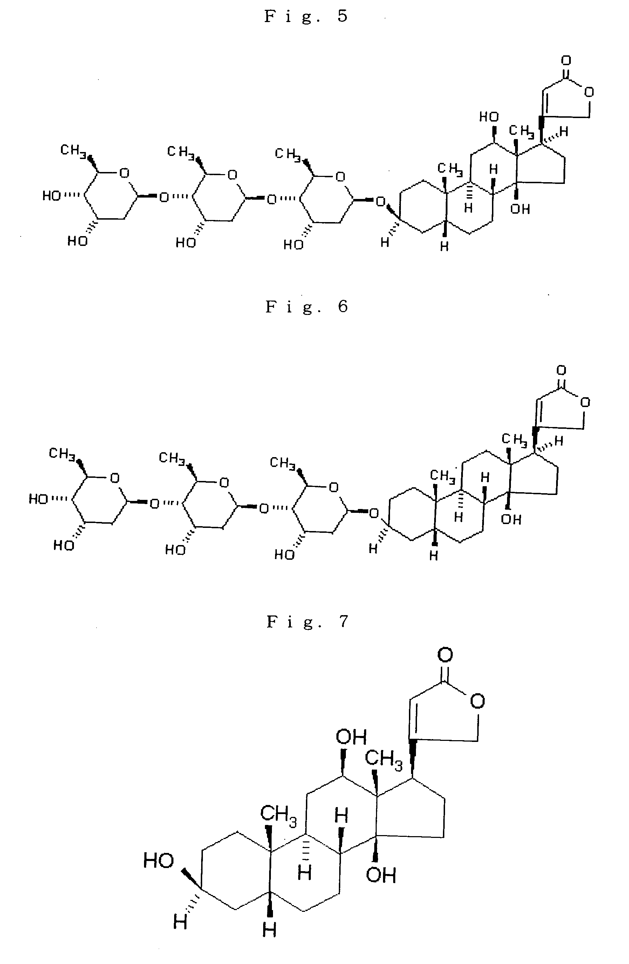

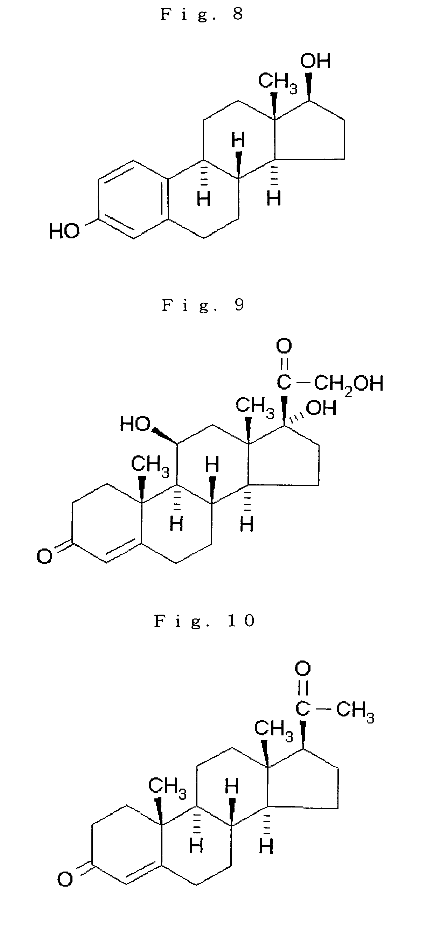

Alkane thiol having only polyethylene glycol and not having the photoreactive group was used as a dummy linker (FIG. 2). An ethanol solution of 20 µM of the photo linker and 980 µM of the dummy linker was prepared, and the gold deposited chip was immersed in the solutions for 24 hours to obtain the surface on which the photolinker was present in the dummy linker (FIG. 3). After washing the substrate surface with ethanol and water, 10 mM DMSO solutions of cyclosporin A (CsA: Mw=1202.61: Fig 4), digoxin (DIG: Mw=780.94: FIG. 5), digitoxin (DTN: Mw=764.94: FIG. 6), digoxigenin hydrate (DXH: Mw=390.51: FIG. 7), β-Estradiol (EST: Mw=272.36: FIG. 8), Hydrocortisone (HCN: Mw=362.46: FIG. 9), and Progesterone (PGS: Mw=314.46: FIG. 10) were spotted using MultiSPRinter automated spotter (supplied from Toyobo Co., Ltd.). For spotting, a flat pin of φ 200 µm was used. DMSO was evaporated without particularly heating during the spotting.

The light at 4 J/cm² from an electric bulb of 365 nm was irradiated using Stratalinker1800 (supplied from Stratagene) to the substrate on which the small molecular compound had been spotted. The irradiation took 45 minutes. The photoreacted substrate was washed with DMSO and ethanol.

A small molecular array formed on the metal substrate was set in an SPR imaging instrument (MultiSPRinter supplied from Toyobo Co., Ltd.), the buffer for measurement of 10mM Hepes[pH7.2], 150 mM NaCl, 0.005% Tween 20 was run in a flow cell.

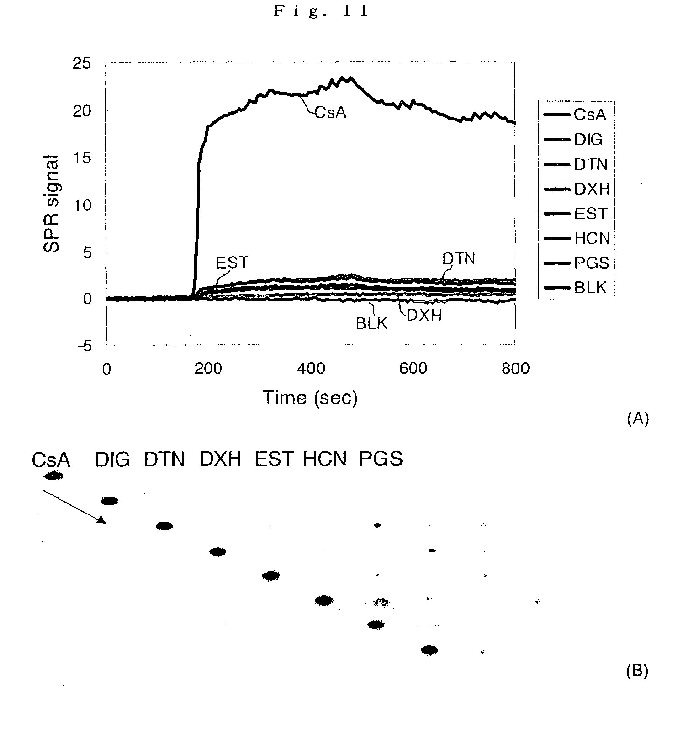

The changes of signals when 50 µg/mL anti-CsA antibody (supplied from Hytest) in the buffer for measurement was run for 5 minutes after confirming that the signals from SPR were stabilized are shown in FIG. 11(A). Differences of images before and after anti-CsA was run are shown in FIG. 11(B). This way, it is obvious that anti-CsA is absorbed to only spots to which CsA was immobilized. This way, the specific binding to the small molecule immobilized on the surface could be observed.

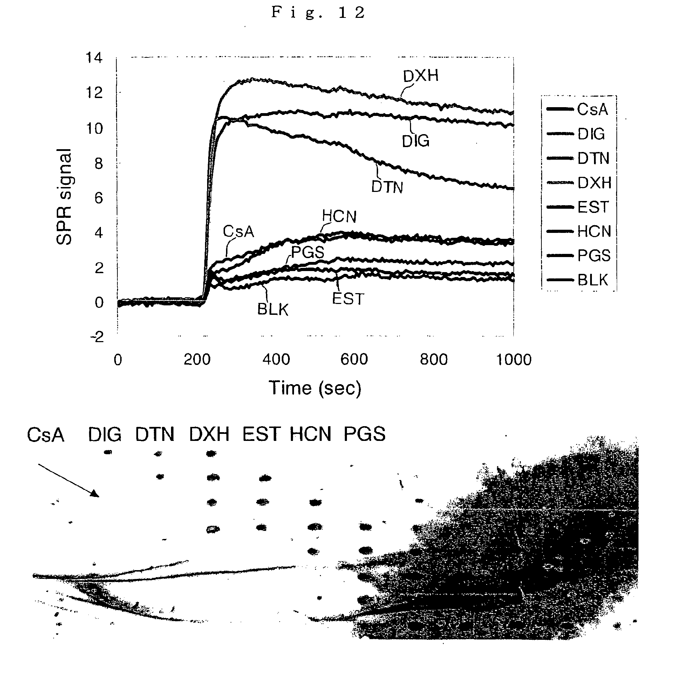

The results when 50 µg/mL anti-DIG antibody (supplied from Sigma) was run are shown in FIG. 12. Although non-specific binding was observed in lower right on the array, the bindings of anti-DIG to DIG, DTN and DXH were obvious.

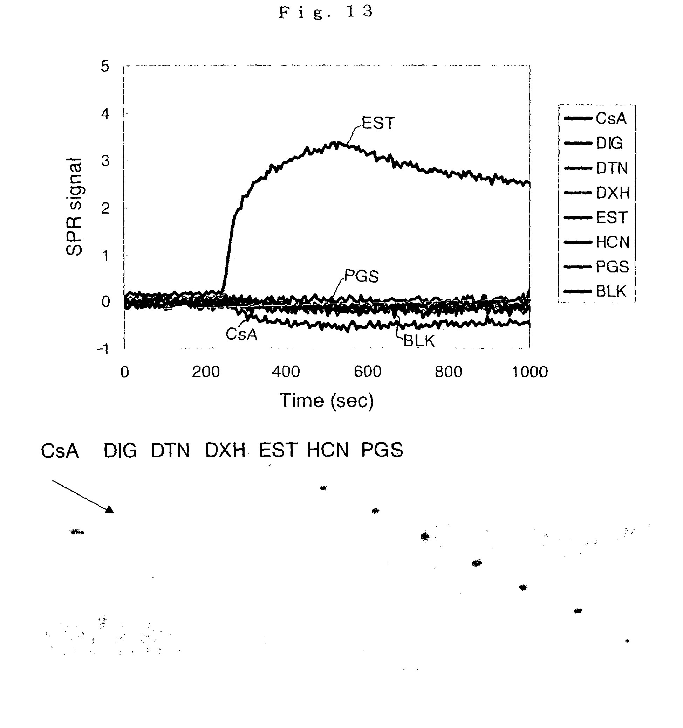

The results when 50 µg/mL anti-EST antibody (supplied from Fitzgerald) was run are shown in FIG. 13. It could be observed that anti-EST was bound to only the spots of EST. No binding to HCN and PGS similar to EST in structure was observed, and the results in which the specificity was very high were obtained.

It was also possible to recycle and use the small molecular array of the present invention. It can be speculated that the interaction of the small molecule with the protein is dissociated by running 10 mM HCl solution and another screening can be done by running another protein.

This way, it was possible to immobilize the small molecular compound on the metal substrate by the photoreaction and observe the specific interaction with the protein by the SPR imaging method. The present invention can be expected to be effective for screening of the small molecular drugs.

### [Comparative Example 1]

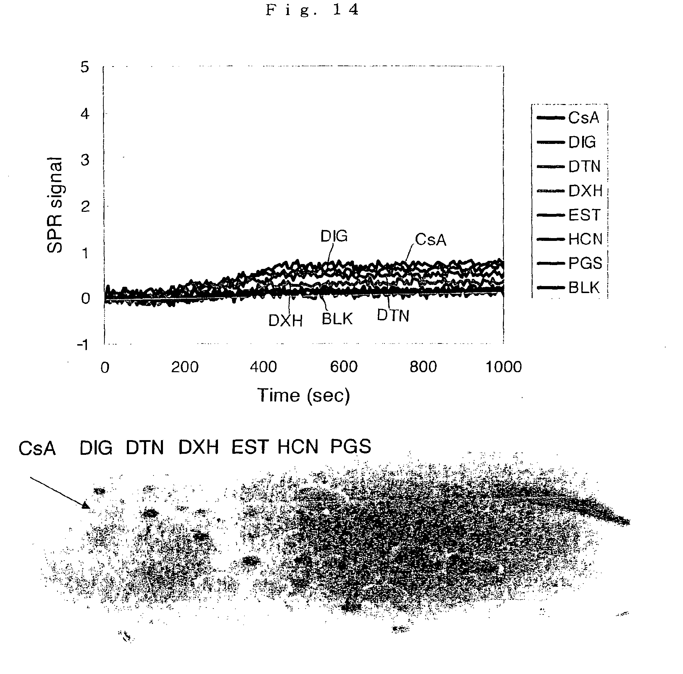

The photolinker and the dummy linker were introduced on the gold deposited chip, and the 10 mM DMSO solutions of seven small molecular substances (CsA, DIG, DXH, EST, HCN, PGS) were spotted in the same way as in Example. Thereafter, the chip was left stand for one hour, and the SPR measurement was performed without irradiating the light. The results when 50 µg/mL anti-CsA antibody in the buffer for measurement was run for 5 minutes are shown in FIG. 14. In this case, the binding of anti-CsA was scarcely observed. It is thought that CsA was not immobilized because the light was not irradiated.

### [Comparative Example 2]

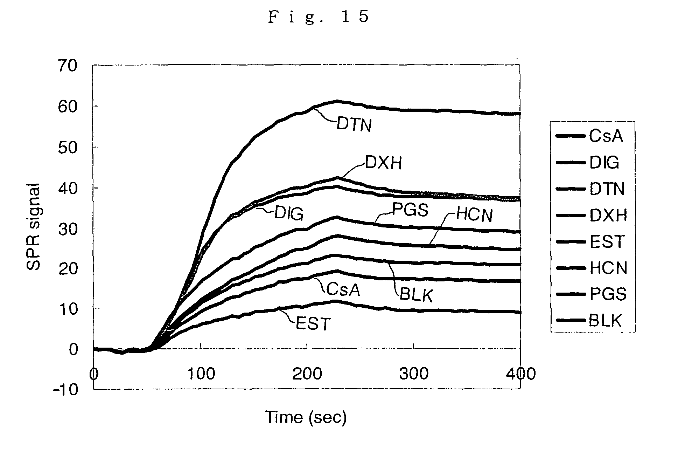

The photolinker and the dummy linker were introduced on the gold deposited chip, and the 10 mM DMSO solutions of seven small molecular substances (CsA, DIG, DXH, EST, HCN, PGS) were spotted in the same way as in Example. Subsequently, the light at 4 J/cm² from an electric bulb of 245 nm was irradiated using Stratalinker1800 (supplied from Stratagene). The irradiation took 40 minutes. The photoreacted substrate was washed with DMSO and ethanol. The SPR measurement was performed using the resulting small molecular array. The results when 50 µg/mL anti-DIG antibody in the buffer for measurement was run for 5 minutes are shown in FIG. 15. In this case, although the bindings of anti-DIG to DIG, DTN and DXH were observed, many bindings to other spots were also observed, and the bindings to blank spots where nothing should have been immobilized were at a not negligible level. It is thought that by irradiating the UV light with a wavelength of 245 nm, the gold-sulfur bond was broken and the gold was exposed, resulting in increased non-specific absorption.

### INDUSTRIAL APPLICABILITY

According to the present invention, the array where the small molecule has been immobilized on the substrate can be obtained, and it is possible to analyze the interaction with the biomolecule.

## Claims

1. A method of label-free observing an interaction, **characterized in that** a substance is immobilized on a metal substrate via a covalent bond by a photoreaction in a method of label-free observing the interaction of a biomolecule with the substance immobilized on the metal substrate.

2. The method according to claim 1 wherein the photoreaction is performed using light with a wavelength of 300 nm or more.

3. The method according to any of claims 1 to 2 wherein a method of observing the interaction is a surface plasmon resonance.

4. The method according to any of claims 1 to 2 wherein a method of observing the interaction is a surface plasmon resonance imaging.

5. The method according to any of claims 1 to 4 wherein multiple substances are immobilized.

6. The method according to any of claims 1 to 5 wherein the immobilized substance is a drug or a drug candidate substance.

7. The method according to any of claims 1 to 6 wherein a molecular weight of the immobilized substance is 2,000 or less.

8. The method according to any of claims 5 to 7 wherein the biomolecule is a protein.
